# EUROPEAN PATENT APPLICATION

(11) **EP 1 882 479 A1**
(43) Date of publication of application: **30.01.2008**
(21) Application number: 07252958.9
(22) Date of filing: 26.07.2007
(51) Int. Cl.: A61L 2/07, A61L 2/24, A61L 2/28

(54) **Autoclaves**

(30) Priority: 28.07.2006 GB 0614988
(71) Applicant: Eschmann Holdings Limited, Lancing, West Sussex BN15 8TJ (GB)
(72) Inventor: Golder, Roger Francis, Ely, Cambridge, CB6 3PQ (GB); Copeland, Terence George, Bexhill on Sea, East Sussex, TH40 2RJ (GB)
(74) Representative: Wilson, Alan Stuart

(57) **Abstract**

An autoclave comprises an enclosure 10 defining a chamber 12, steam supply means 20, 22 arranged to supply steam to the chamber, steam venting means 34, 40, 58, 60 arranged to vent steam from the chamber, and control means 100, 102 arranged to control operation of the autoclave. The control means comprises a first controller 100 and a second controller 102, each controller being arranged to define a respective set of conditions required for a change of state of the autoclave, to monitor a parameter of the autoclave and determine therefrom whether its respective conditions have been met, and the control means is arranged to allow the change of state only if both sets of conditions have been met.

## Description

The present invention relates to autoclaves, and in particular to control systems for autoclaves.

Autoclaves are used in a large number of applications, and in many of these it is important to ensure that the autoclave is operating correctly. For example, in medical applications it is essential that, for example, surgical devices and dental tools are properly sterilized. Various methods of testing the operation of an autoclave are known, but these generally comprise running test cycles which are separate from any normal operational cycles. This takes up time preventing use of the autoclave, and also does not check every operational cycle, so the autoclave may run a number of cycles before any fault is detected. There is therefore an ongoing need to improve the reliability of autoclaves.

Accordingly the present invention provides an autoclave comprising an enclosure defining a chamber, steam supply means arranged to supply steam to the chamber, a control system arranged to control operation of the autoclave, wherein the control system comprises control means and monitoring means, the control means and monitoring means each being arranged to define a respective set of conditions required for a change of state of the autoclave, and the control system is arranged to allow the change of state only if both sets of conditions have been met.

The change of state may be to a 'passed' state indicating that a sterilization cycle of the autoclave has been correctly completed. The control means may include an arbiter arranged to receive inputs from each of the first and second controllers and to allow the autoclave to move to the 'passed' state only if both controllers have determined that their respective set of conditions has been met.

The control means and the monitoring means may each be arranged to monitor at least one parameter, and to determine therefrom whether its respective conditions have been met. For example the at least one parameter may be one or more of temperature, pressure and time. For example they may monitor temperature or pressure of the chamber (or some other part of the system), or both, as a function of time, or they may measure temperature or pressure, or both, at predetermined times.

The present invention further provides an autoclave comprising an enclosure defining a chamber, steam supply means arranged to supply steam to the chamber, a temperature sensor arranged to produce a signal that varies with the temperature in the chamber, a pressure sensor arranged to produce a signal that varies with the pressure in the chamber and control means arranged to receive the signals from the temperature sensor and the pressure sensor, to determine whether the relationship between the two signals meets a predetermined condition, and produce an error output if the condition is not met.

Preferred embodiments of the present invention will now be described by way of example only with reference to the accompanying drawings in which:
**Figure 1** is a schematic diagram of the pressure circuit of an autoclave according to an embodiment of the invention;
**Figure 2** is a functional block diagram of the control system of the autoclave of Figure 1;
**Figure 2a** is a diagram of an arbiter forming part of the control system of the autoclave of Figure 1;
Figure 3 is a diagram showing a door locking system of the autoclave of Figure 1;
Figure 4 is a diagram of a protective system of the autoclave of Figure 1;
Figure 5 is a functional block diagram of a protective controller of Figure 4;
Figure 6 is a state diagram showing various states of the autoclave of Figure 1;
Figure 7 is a flow chart showing operation of the system in a Test state;
Figure 8 is a flow chart showing operation of the system in an Idle state;
Figure 9 is a flow chart showing operation of the system in a Sterilize state.

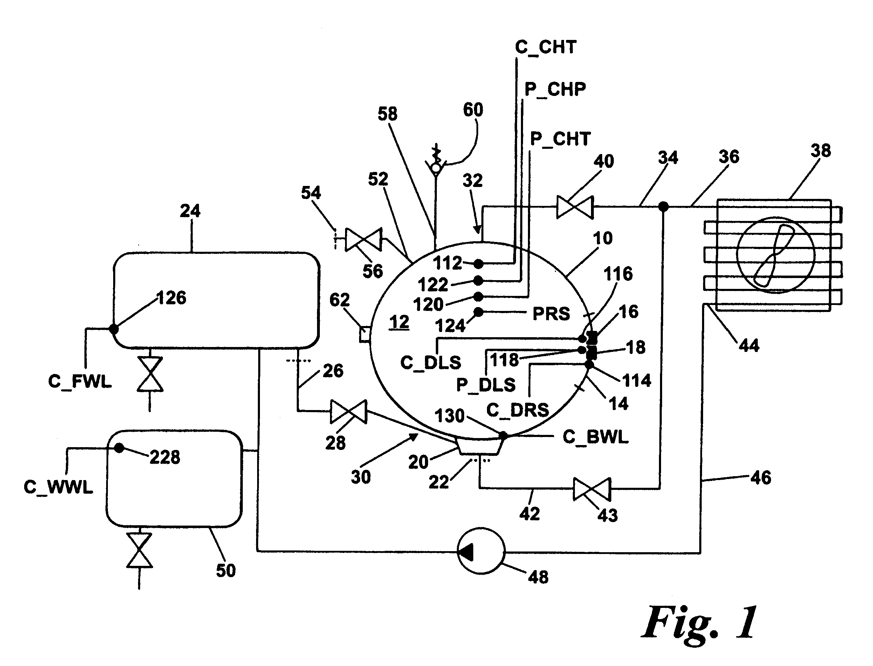

Referring to Figure 1, an autoclave comprises an enclosure 10 defining a chamber 12. The enclosure 10 includes a closure, for example in the form of a door 14 which can be locked independently by each of two door locks 16, 18. A boiler 20 is provided at the bottom of the chamber 12, and opens into the chamber 12. A heater 22 is arranged to heat water in the boiler 20 so that it boils and evaporates, thereby providing a supply of steam to the chamber 12.

A feed water tank 24 provides a source of water and is connected to the chamber 12 by a water feed pipe 26. A feed water valve 28 is arranged to control the flow of water from the feed water tank 24 to an inlet 30 in the boiler 20. A steam outlet 32 in the top of the chamber 12 is connected via an outlet pipe 34 to the inlet 36 of a condenser 38. A chamber vent valve 40 is arranged to control the flow of steam through the vent pipe 34 to the condenser 38. A boiler drain pipe 42 connects the bottom of the boiler 20 to the inlet 36 of the condenser 38 to enable the boiler 20 to be drained if required, under control of a drain valve 43 in the drain pipe 42. The outlet 44 of the condenser 38 is connected via a return pipe 46 to the feed water tank 24. A pump 48 is provided in the return pipe 46 to pump condensed water from the condenser 38 back to the feed water tank 24. A waste water tank 50 is also connected to the return pipe 46 to collect condensed water that is not returned to the water feed tank 24. In other embodiments this waste water tank 50 can be omitted and the water returned to the feed water tank 24.

The chamber 12 has an air inlet 52 which is connected to atmosphere via an air filter 54 and an air inlet valve 56, which is arranged to control the inlet of air to the chamber 12. The chamber 12 also has a safety air outlet 58 that vents to atmosphere via a safety valve 60, which is arranged to open if the pressure in the chamber 12 exceeds a predetermined level. An electric heater in the form of a band heater 62 is provided to heat the chamber 12.

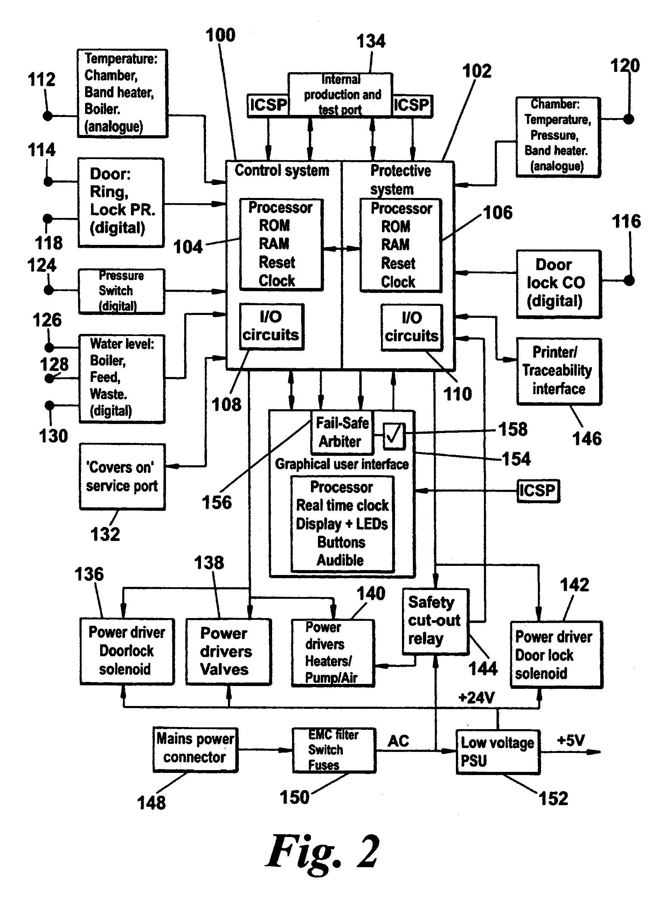

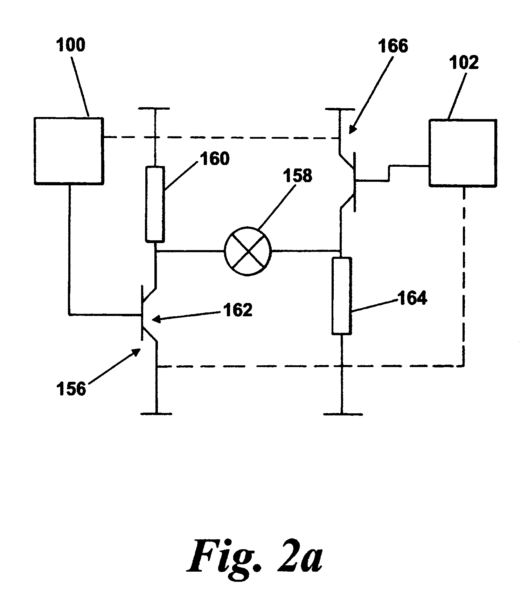

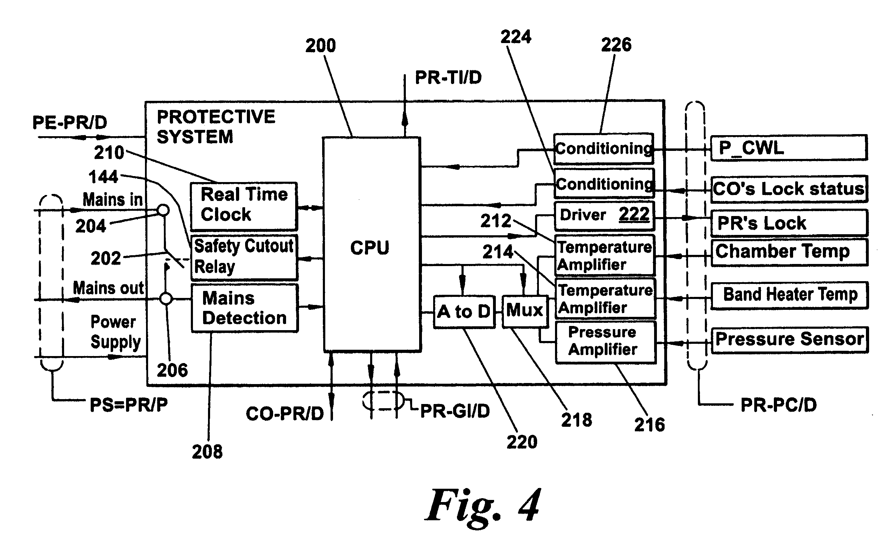

Referring now also to Figure 2, the autoclave is controlled by a control system comprising a controller 100 and a monitoring system in the form of a protective system 102, each of which comprises its own respective processor, ROM, RAM and clock, collectively indicated as 104 and 106, and its own I/O circuits 108, 110. These features of the protective system 102 are described in more detail below with reference to Figure 4.

A first temperature sensor 112 produces an output signal C_CHT indicative of the temperature in the chamber, which is input to the controller 100, together with further signals indicative of the band heater temperature and boiler temperature. A door closure sensor 114 produces a signal C_DRS indicative of whether the door 14 is open or closed, and that is also input to the controller 100. A first door lock sensor 116 produces a signal C_DLS indicative of the state of the first door lock (or control door lock) 16, which is input to the protective system 102. A second door lock sensor 118 produces a signal P_DLS indicative of the state of the second door lock (or protective door lock) 18, which is input to the controller 100. A second chamber temperature sensor 120 produces an output signal P_CHT indicative of the temperature in the chamber, which is input to the protective system 100. A chamber pressure sensor 122 produces an output signal P_CHP indicative of the pressure in the chamber, which is input to the protective system 100. An independent second chamber pressure sensor 124 in the form of a pressure switch is arranged to cut the drive signal to the door lock actuator for the first door lock 16 if the chamber pressure exceeds a predetermined maximum. This additional safety feature ensures that the chamber door cannot be opened if the chamber pressure is above this maximum safe pressure. Water level sensors 126, 128, 130 in the feed water tank 24, waste water tank 50 and boiler 20 respectively are arranged to produce signals C_FWL, C_WWL and C_BWL that are indicative of the water level in the feed water tank 24, waste water tank 50 and boiler 20 respectively. Those signals are input to the controller 100. A 'covers on' service port 132 provides test and diagnostic accesses for service technicians to the controller 100. An internal and production test port 134 provides access to the controller 100 and protective system 102 for production and test purposes.

The controller 100 provides door lock control signals to a power driver 136 for the solenoid of the first door lock 16 to control locking and unlocking of the first door lock, a power driver 138 for the system's valves which can open and close them, and a power driver 140 for the system's heaters and pumps to turn them on and off. The protective system 102 provides control signals to a power driver 142 for the solenoid of the second door lock 18 to control locking and unlocking of the second door lock, and to a safety cut-out relay (SAF) 144 which can cut the power to the power driver 140 in the event of a fault. The protective system 102 provides an output to a printer/traceability interface 146. This enables it to output a record of the operation of the autoclave.

Power is provided to the autoclave from a mains power connector 148, via an EMC filter, switch and fuses, collectively indicated as 150, which provide mains AC power to the power driver 140 via the safety cut-out relay, and to the power drivers 136, 138, 142 for the valves and door locks via a low voltage power supply unit 152.

The autoclave also includes a graphical user interface (GUI) 154. This includes a processor, real time clock, display and LEDs to provide visual feedback to a user, buttons to provide a user input, and a speaker to provide audible feedback to the user. Both the controller 100 and protective system 102 can receive inputs from the GUI 154, and the controller 100, and to a lesser extent the protective system,_can produce outputs to the GUI to produce feedback to the user in the form of information and prompts to prompt the user to carry out certain operations. The GUI 154 also includes a fail-safe arbiter 156, which is arranged to indicate to a user when a sterilization cycle has been performed correctly, i.e. when the cycle has passed, based on signals from the controller 100 and protective system 102. The GUI includes an indicator LED 158 arranged to be lit when a cycle has passed.

As shown in Figure 2a the arbiter 156 comprises a first resistor 160 connected to a supply voltage and connected in series via a first switch 162 to ground, and a second resistor 164 connected in series via a second switch 166 to the supply voltage and directly to ground. The indicator LED 158 is connected between the bottom end of the first resistor 160 and the top end of the second resistor 164. The resistors 160, 164 are sufficiently high that the LED will only be lit if both the switches 162, 166 are closed. The controller 100 is arranged to output a pass signal if it determines that the cycle has been passed, which is arranged to close the first switch. The protective system 102 is arranged to output a pass signal if it determines that the cycle has been passed, which is arranged to close the second switch. Therefore only if both the controller 100 and the protective system 102 determine from their respective inputs that the cycle has been passed will both switches be closed and the indicator LED lit to indicate to a user that the cycle has been completed successfully.

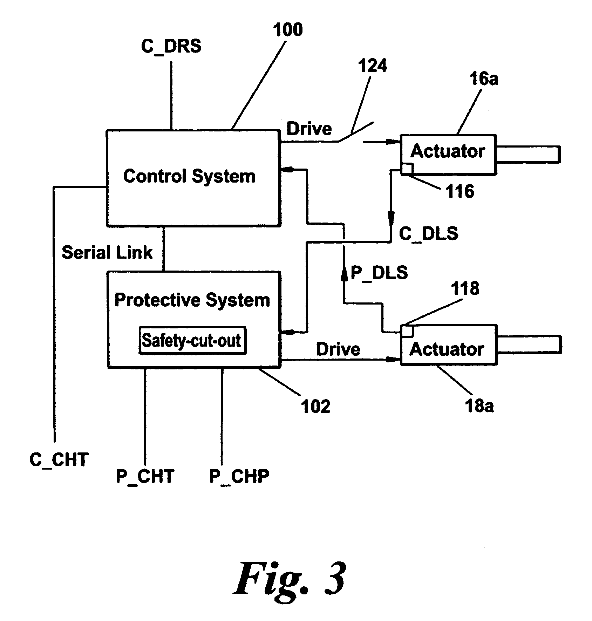

The door locking system, which is included in Figures 1 and 2, is more clearly shown in Figure 3, from which it can be seen that the controller 100 sends a drive signal to the actuator 16a of the control door lock 16, and receives the signal P_DLS indicative of the position of the actuator 18a of the protective door lock 18, and the protective system 102 sends a drive signal to the actuator 18a of the protective door lock 18, and receives the signal C_DLS indicative of the position of the actuator 16a of the control door lock 16. The fact that each of the CO 100 and the PR 102 controls one of the locks and monitors the other of the locks provides a secure check that the door is locked as required.

Referring to Figure 4, the protective system 102 comprises a processor 200 arranged to control the safety cut-out relay 144, which in turn controls the opening and closing of a switch 202 between a mains-in terminal 204 and a mains-out terminal 206. The protective system 102 further comprises a mains detection module 208 arranged to check operation of the safety cut-out relay, and a real time clock 210 arranged to provide timing signals to the processor 200. First and second temperature amplifiers 212, 214 are arranged to receive temperature signals from the chamber temperature sensor 120 and a band heater temperature sensor, and a pressure amplifier 216 is arranged to receive the pressure signal from the chamber pressure sensor 122. A multiplexer 218 is arranged to receive the amplified signals from the three amplifiers 212, 214, 216 and input them to an analogue-to-digital converter 220, which inputs the converted digital signals to the processor 200. A protective lock driver 222 is arranged to drive the protective lock actuator 18a under control of the processor 200. Signal conditioning modules 224, 226 are arranged to receive signals from the control lock position sensor 116, and optionally a further water level sensor, and condition them before inputting them to the processor 200.

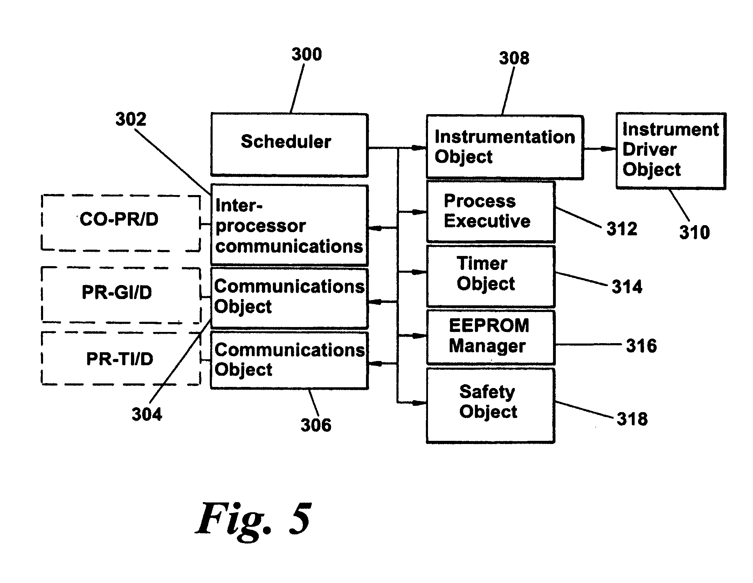

The software of the protective system 102 is written in an object-oriented manner so as to provide code segregation to improve comprehension and analysis, and to permit predictable behaviour after rework. Referring to Figure 5, the objects included in the protective system software include a scheduler module 300 for controlling background tasks, in inter-processor communications module 302 arranged to control communications between the protective system 102 and the controller 100, a further communications module 304 arranged to control communications between the protective system 102 and the graphical interface 154, a further communications module 306 arranged to control communications between the protective system 102 via the printer/traceability interface 146 with a traceability system, an instrumentation module 308 arranged to control the collection of data from the sensors and other instrumentation that the protective system communicates with, and an instrumentation driver module 310 arranged to control operation of the instrumentation, a process executive 312 arranged to monitor, evaluate and check the operation of the autoclave, a timer module 314, an EEPROM manager module 316, and a safety module 318 arranged to monitor operation of the autoclave to ensure that it is safe, and take appropriate action if it detects any conditions that render the system unsafe.

During operation of the autoclave the controller 100 and the protective system 102 each move between various control states. They also communicate with each other over a serial link so that each can determine the current state of the other and the states that they are in can be coordinated. They also communicate to each other over the serial link the measurements and readings that they receive from the various sensors, and the results of all tests and checks that they carry out during operation of the autoclave. This enables each of them to check whether various conditions are met to enable them to change state, or to cause them to change state, or to enable them, or cause them, to remain in their current state. In general the states of the controller 100 and protective system 102 are coordinated so that they are both in the same state, and therefore the state that they are both in can be considered as the state of the autoclave as a whole. Therefore the state of the autoclave as a whole can, in many cases, only change from one of the states to another if both the controller 100 and the protective system 102 agree on the new state. If they are in different states, then this may be transitory, or it may be indicative of a fault. Generally at transitions from one state to another each of the CO 100 and PR 102 checks that its conditions for the transitions have been met, then enters the new state, and then checks that the other has entered the new state. Only then do they both determine that the system as a whole has entered the new state and continue with the operations appropriate to that state.

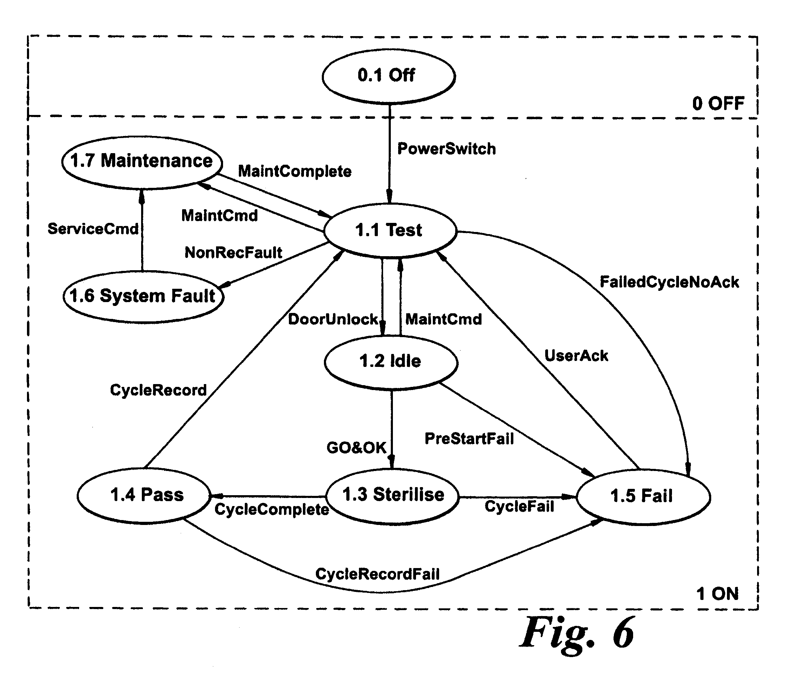

The main states of the autoclave will now be described with reference to Figure 6. When the power supply to the autoclave is switched off, the autoclave is in an Off state 0.1. From there, when the power is switched on, the autoclave enters a Test state 1.1, in which various tests are carried out that will be described in more detail below. From the Test state 1.1 the autoclave can move to an Idle state 1.2, for example if the door 14 is unlocked after completion of the relevant tests. From the Idle state 1.2, if instructions are input via the GUI 154 to start a sterilisation cycle and various conditions are met, then the autoclave moves to a Sterilise state 1.3 in which the sterilising process is carried out. If the sterilising cycle is successfully completed, then the autoclave moves to a Pass state 1.4. From the Pass state, if a record of the cycle is successfully recorded, the autoclave moves back to the Test state 1.1. However, if the record is not successfully recorded, the autoclave moves to a Fail state 1.5. The autoclave also moves to the Fail state 1.5 from the Sterilise state 1.3 if the cycle is not successfully completed, and from the Idle state 1.2 if pre-start checks fail, and from the Test state 1.1 if a cycle is failed and no user acknowledgement if received via the GUI 154. If acknowledgement is subsequently received, the process executive returns to the Test state. From the Test state 1.1 if a non-recoverable fault occurs, then the autoclave enters a System Fault state 1.6. From either the Test state 1.1 or the System Fault state 1.6, if a service command is received via the service port 132, then the autoclave enters a Maintenance state 1.7. From there, when maintenance is complete, the autoclave returns to the Test state 1.1.

In each state the CO 102 and PR 100 perform a number of operations and generally it is a requirement that certain functions must be performed and certain checks made before that state can be entered. Also while this clearly applies to the states of Figure 6, which can be considered as control states, it also applies to other states of the autoclave, including physical states such as the state of the door, i.e. whether it is open or closed, locked or unlocked, and states of the chamber, e.g. whether it is pressurised or not or heated or not.

During the sterilizing cycle the boiler heater 22 is turned on and steam begins to flow slowly increasing the temperature of the chamber 12. Once a significant amount of steam begins to enter and condense in the condenser 38, the back-pressure generated by this increases the chamber pressure up to an equilibrium level. Chamber temperature then continues to increase as the proportion of steam in the chamber mix increases towards saturation. When the chamber reaches saturation it equilibrates at the pressure caused by the flow through the condenser and the associated saturated steam temperature. The temperature and pressure in the chamber therefore level off. At this point further heating does not affect the temperature or pressure within the chamber 12. Then eventually the cycle moves into a phase of increasing pressure, and the temperature and pressure increase in proportion based on the relationship determined by steam saturation. The cycle includes a pre-conditioning phase in which the temperature and pressure are increased and decreased in a controlled manner in order to ensure that steam reaches all parts of the equipment to be sterilised. It then enters a sterilisation phase in which the temperature and pressure are held constant at a plateau for a predetermined hold time. The final phase of the cycle is a post conditioning phase during which cooling and drying are carried out. The controller 100 has the temperatures, pressures and timings that are required through the cycle programmed into it and controls the various components of the system to ensure that the cycle is followed. The controller 100 and protective system 102 both check various parameters of the cycle, which may be the same parameters for them both or may be different, to check whether the cycle has been successfully completed or not. These parameters include the temperature and pressure of the chamber and the times at which they are reached and the times for which they are maintained. More specifically these checks are carried out at predetermined times or waypoints in the cycle. Each of the controller 100 and protective system 102 determine when these times occur using their respective clocks. They then each check the chamber temperature using their respective temperature sensors 112, 120, the protective system 102 checks the chamber pressure using the pressure sensor 122, and communicates the measured pressure to the controller 100. The controller 100 checks that the relationship between the measured pressure and measured temperature meets predetermined criteria, as expected under saturated steam conditions. Provided all of these measurements are in agreement then the waypoint is deemed to have been reached. If any of the waypoints is not reached, i.e. the temperature and pressure are not confirmed as correct by the controller 100 and the protective system 102, then the cycle is deemed to have failed and the system enters the fail state.

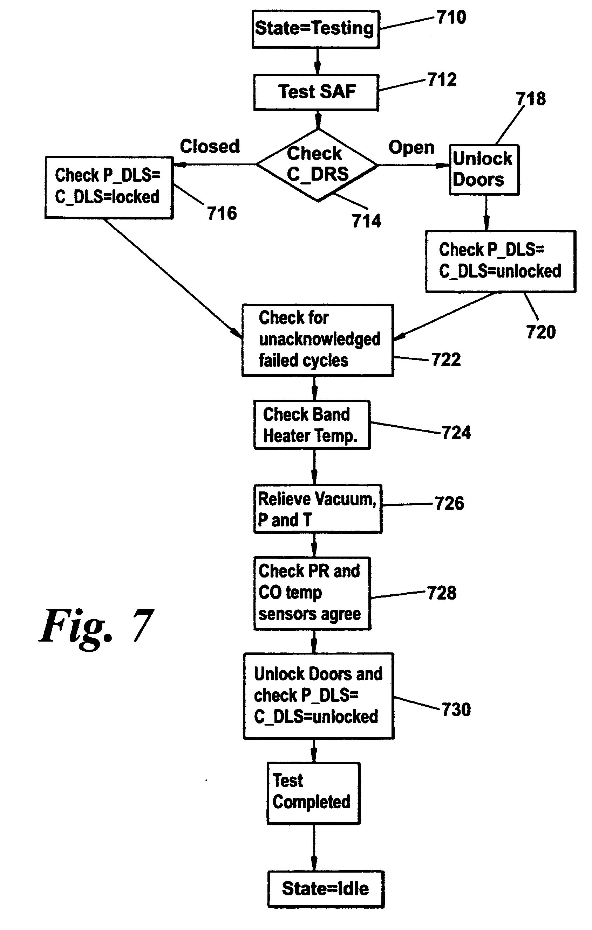

Referring to Figure 6, when the system is switched on it first enters the test state 1.1. Referring to Figure 7, at step 710 the CO 100 and the PR 102 both enter the test state and, because each can continually monitor the state of the other, each checks that the other has entered the test state. When this is confirmed, the PR 102 checks the safety cut-out relay at step 712. Assuming that the cut-out relay test is positive, the system proceeds to step 714 where both the CO 100 checks from the data communicated over the serial link by the PR, and the PR 102 checks directly from the P_DRS signal from the door sensor, whether the door is open. If the door is determined to be closed, then the PR 102 and the CO 100 both check that both of the door locks are locked from the P_DLS and C_DLS signals at step 716. If they are, then the system proceeds to step 722 where it checks for any unacknowledged failed cycle signals.

If at step 714 the CO 100 and PR 102 determine that the door is open, the system proceeds to step 718 where the CO 100 unlocks the first door lock 16 and the PR 102 unlocks the second door lock 18. When the appropriate control signals have been sent to the drivers for the door lock actuators to cause this to happen, the CO 100 and PR 102 check at step 720 that the locks are indeed both locked from the P_DLS and C_DLS signals, and provided they are, the system proceeds to step 722 where it checks for any unacknowledged failed cycle signals. The reason for this is as follows. Where the system has entered the fail state, normally a user has to input an acknowledgment signal via the GUI 154 before the system can leave the fail state. However, if the system has been switched off when in the fail state, an unacknowledged failed cycle indicator may still be recorded in memory. If such an indicator is detected, the system will return to the fail state until an appropriate acknowledgement is input by the user. This prevents access to a non-sterile load without the proper acknowledgement.

From step 722, if no unacknowledged failed cycles are detected, then the system proceeds to step 724 where the CO and PR both check that the band heater temperature is below a safe threshold, in this case 55°C. Both the PR & CO detect this from their sensor signals. If it is, then the system proceeds to step 726 where the CO takes the appropriate steps to relieve any vacuum or pressure in the chamber and allow the temperature in the chamber to reach a safe and appropriate temperature, and to maintain a suitable water level in the boiler. In this case the band heater and boiler heater are turned off, the water supply valve 28 is closed, the drain valve 43 is opened, and the air inlet valve 56 is opened. The system then proceeds to step 728 where the CO and PR check the temperature of the chamber using the signals from the respective temperature sensors 112, 120, and then check with each other that the two temperature sensors agree and both indicate the same chamber temperature. Provided that they do, the system proceeds to step 730 where the CO and PR each unlock their respective door locks 16, 18 and then each check from the signals from the door lock sensors 116 118 that the door locks have indeed been unlocked. Provided they have, the test is completed, and the CO and PR both enter the idle state.

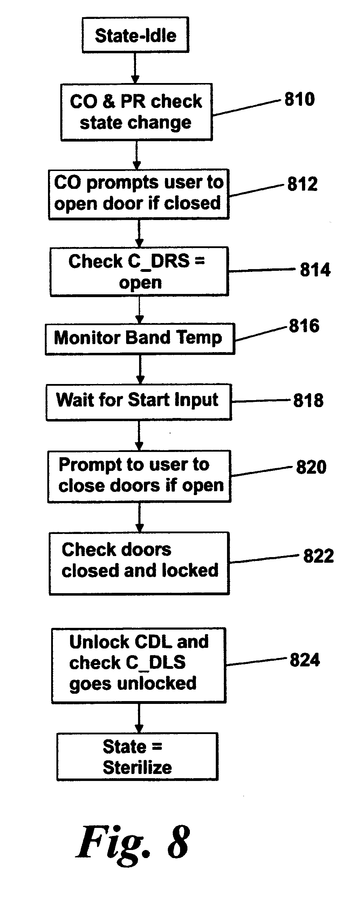

Referring to Figure 8, on entering the idle state, the CO 100 and PR 102 both check at step 810 that the other has entered the idle state. If they both have, then the system proceeds to step 812 where the CO 100 issues a prompt via the GUI to the user to open the door, if the door is closed. Then at step 814 the PR 102 checks from the C_DRS signal that the door is open. If, or when, the door is detected as being open, the system checks the temperature of the band heater at step 816 and then waits at step 818 for an input from a user via the GUI to start a sterilization cycle. When the instruction to start is input at step 818, the system progresses to step 820 where the CO 100 checks whether the door is open, and, if it is, prompts the user, by issuing a prompt via the GUI, to close the door. When the PR detects, from the C_DRS signal that the door is closed, the CO and the PR lock their respective door locks at step 822. A final check of the safety relay 144 & the door-lock pressure switch is then carried out at step step 824. To do this, the first door lock is unlocked by the CO 100, and the PR 102 checks that the first door lock is unlocked from the C-DLS signal. If it is not, this indicates a fault in the door-lock pressure switch and the system goes to the fail state. However, assuming this test is passed, the CO and PR each lock their door locks and each check that the door lock controlled by the other has been locked. Provided the door locks are both successfully locked and checked, the door is deemed to be in a locked state and the CO and PR move to the sterilize state and the sterilization process is carried out.

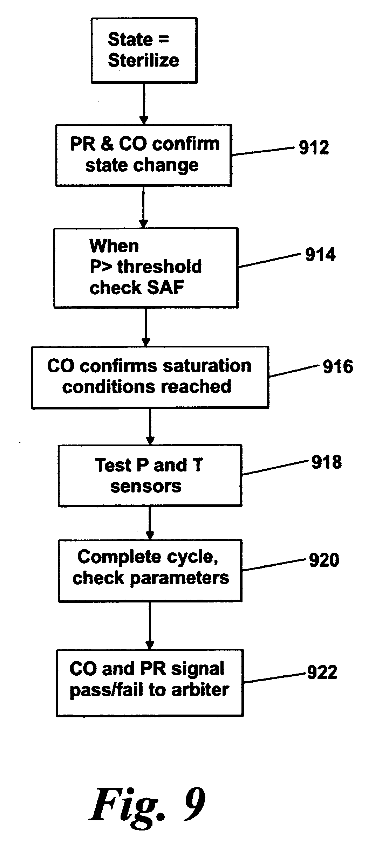

Referring to Figure 9, on entering the sterilization state, at step 912 the CO 100 and PR 102 both change their state to the sterilization state and check that the other has also changed state. Provided this check is passed, the CO starts at step 914 to control the various sub-systems of the autoclave to start the cycle. As the pressure in the chamber increases, the signal from the pressure sensor 122 is monitored by the PR 102. When it exceeds the threshold of the pressure switch 124, the PR 102 communicates this to the CO 100 which first checks that the PDL_S signal indicates that the second door lock is locked, and, if it is, communicates this to the PR 102, which initiates a check of the pressure switch 144. To perform this check, the PR 102 issues an unlock signal to the door lock driver to unlock the second door lock. The CO monitors the second door lock sensor signal, and, if it does not indicate that the second door lock is unlocked, this confirms that the pressure switch has cut the power to the door locks as it should. Then the cycle continues until the CO determines that the point in the cycle has been reached where steam saturation should have occurred in the chamber, at which point it communicates this to the PR 102 at step 916. In response to this, at step 918 the PR 102 checks the pressure in the chamber as indicated by the pressure sensor 122 and the temperature in the chamber as indicated by the two temperature sensors 112, 120. It then checks that the two temperature sensors are in agreement to within appropriate tolerances and also checks that the measured pressure and the measured temperature are related to each other in the manner expected for saturated steam conditions. This checks that the temperature and pressure sensors are all working.

During the rest of the cycle, indicated as step 920 the CO 100 and PR 102 both continue to monitor the measurable cycle parameters, in this case temperature and pressure reached at each stage, and the length of time taken to reach each stage, and the length of time for which each stage is maintained, to check that they meet predetermined conditions. Each of the CO 100 and PR 102 independently determines whether the conditions have been met and therefore whether the cycle has been passed or failed, and indicates this by means of an arbiter signal to the arbiter 156 at step 922. The arbiter monitors the arbiter signals from both the CO and the PR and if they both indicate that the cycle has been passed, it determines that the cycle has indeed been passed and indicates this to the user via the GUI as described above. If either the CO or the PR determines that the cycle has not been passed, then one of the required signals will not be sent to the arbiter and the arbiter will not indicate a pass via the indicator LED 158.

During operation of the autoclave as described above, if any of the checks does not result in the expected outcome as required for a successful cycle, then the system goes to the fail state. For example if the CO and the PR do not agree, or at least do not agree within a predetermined time limit, on the state in which the system should be, or if they do not agree on the measurements of any of the measurable parameters of the systems operation, then the system enters the fail state. As indicated in Figure 4, if the band heater is detected as overheating, then the system is arranged to take appropriate action: a fault is recorded, the door is locked and the power supply is cut off by opening the safety cut-out relay 202.

A further check carried out during operation of the system is for a service connection having been made to the system, as indicated by the presence of an 'active service' flag. If a service connection is detected, then the system enters the 'service' state and waits for the service technician to input the appropriate authorisation code to clear the active service flag.

Referring back to Figure 5, the process executive 312 for the PR 102 is concerned with the evaluation of the process when the autoclave is in a normal operating mode. It is responsible for analysing the sterilising cycle based on the type or cycle selected (received from the GI) and state parameters received over the serial link from the CO. Independently of the CO 100, it assesses the instrumentation, including the various sensors, for integrity, via its safety object 318.

The sequencing for the process executive has several phases. Close cooperation will normally take place between the PR 102 and the CO 100 as described above. Each will maintain a copy of the state that the other is in, which is communicated to it by means of the inter-processor link. The basic idea is that each independently determines whether a change of state should take place, but will not proceed unless both agree.

Whenever there is disagreement, the nature of the disagreement is recorded in memory in the fault log of the PR 102. During a sterilising cycle, the PR 102 is arranged to announce any faults to the user by recording a suitable announcement in the cycle log data sent to the traceability system, and to announce a fault to the arbiter 156 on the GUI 154. The CO 100 is also arranged to record the nature of the disagreement in its fault log, and announce such faults to the user via the GUI 156. In addition, the PR 102 is arranged to maintain in an area of memory set aside for the purpose, an unacknowledged failed cycle error code. This code is cleared to "No Fault" on correct acknowledgement by the user via the GUI 156. In the event of a fault causing either CO 100 or PR 102 to move to a state without agreement, the other will then be able to detect the fault and take the necessary action to safeguard the system. Within the PR 102 there are mainly autonomous objects as shown in Figure 5, and the two objects in particular which work together closely are the process executive 312 and the safety object 318. The process executive communicates with the CO over the serial link and checks the communications are functioning correctly, and also initiates changes of state of the PR. The safety object controls the second door lock and performance of the tests carried out by the PR, such as the test of the pressure switch and safety relay.

It will be appreciated that the embodiments described above monitor their operation during each sterilizing cycle that they perform, and can determine whether they have performed the sterilizing cycle correctly or not, and indicate this to a user. Furthermore, because the controller 100 and protective system 102 both check performance of the cycle, and confirm passing to the arbiter which then determines whether both have determined that the cycle has been passed before indicating to a user that it has been passed, the chances of the arbiter indicating that the cycle has been passed when it has not are exceedingly small. It is therefore expected that the autoclaves described above could operate on a system of parametric release, in which the autoclave system itself checks the parameters of the cycle it has performed and makes the final decision as to whether the cycle has been passed or failed, and no further system checks are required.

It will be appreciated that many modifications can be made to the embodiment described whilst still falling within the scope of the invention. For example, while the controller 100 and protective system 102 are each provided in the form of a single control unit with a processor and associated memory, either of them could include a number of processors either located together or spaced apart in a distributed manner.

## Claims

1. An autoclave comprising an enclosure defining a chamber, steam supply means arranged to supply steam to the chamber, a control system arranged to control operation of the autoclave, wherein the control system comprises control means and monitoring means, the control means and monitoring means each being arranged to define a respective set of conditions required for a change of state of the autoclave, and the control system is arranged to allow the change of state only if both sets of conditions have been met.

2. An autoclave according to claim 1 wherein the change of state is a change of state of a closure of the autoclave.

3. An autoclave according to claim 1 or claim 2 wherein the change of state is a change from a locked state to an unlocked state, or from an unlocked state to a locked state.

4. An autoclave according to claim 3 comprising two locking mechanisms each of which can be switched between a locked state and an unlocked state such that the closure is only unlocked if both locking mechanisms are in the unlocked state.

5. An autoclave according to claim 4 wherein each of the locking mechanisms can be controlled by a respective one of the control means and the monitoring means.

6. An autoclave according to claim 5 wherein each of the control means and the monitoring means is arranged to unlock its respective locking mechanism if its respective set of conditions is met.

7. An autoclave according to claim 5 or claim 6 wherein each of the control means and the monitoring means is arranged to monitor the state of the locking mechanism controlled by the other.

8. An autoclave according to any foregoing claim wherein each of the control means and the monitoring means is arranged to monitor at least one parameter of the autoclave and determine therefrom whether its respective conditions have been met.

9. An autoclave according to claim 8 wherein the at least one parameter is at least one of the temperature and pressure within the chamber, and time.

10. An autoclave according to claim 9 comprising two temperature sensors each arranged to provide an independent measure of the temperature in the chamber to a respective one of the control means and the monitoring means.

11. An autoclave according to any of claims 8 to 10 including a pressure sensor wherein the control system is arranged to use a signal from the pressure sensor as a measure of temperature.

12. An autoclave according to any foregoing claim wherein the control means and the monitoring means each include a respective processor.

13. An autoclave according to any foregoing claim wherein the control means and the monitoring means each include a respective clock.

14. An autoclave according to any foregoing claim wherein each of the control means and the monitoring means is arranged to check the pressure and temperature of the chamber at predetermined times in a sterilization cycle.

15. An autoclave according to any foregoing claim wherein the change of state comprises changing to a state or changing from a state.

16. An autoclave according to any foregoing claim wherein at least one of the states comprises a control state defined by the control means.

17. An autoclave according to any foregoing claim including an arbiter arranged to determine when both sets of conditions have been met and to produce an output indicating that they have.

18. An autoclave according to claim 14 further comprising a user interface arranged to produce a first output if both sets of conditions have been met and a second output if they have not.

19. An autoclave according to claim 17 or claim 18 wherein the arbiter is arranged to produce the output when a sterilization cycle has been completed to indicate that the cycle has met predetermined conditions.

20. An autoclave according to claim 18 wherein each of the control means and the monitoring means is arranged to output a pass signal only if its respective conditions have been met, and the arbiter is arranged to receive the pass signals and output a further pass signal only if it receives pass signals from both the control means and the monitoring means.

21. An autoclave according to any foregoing claim wherein one of the control means and the monitoring means is arranged to define a state that it is in, and to communicate this to the other of the control means and the monitoring means.

22. An autoclave according to claim 21 wherein one of the control means and the monitoring meansis arranged, on changing its state, to check that the other has performed a corresponding change of state.

23. An autoclave according to claim 22 wherein, if there is a disagreement between the control means and the monitoring meansas to the state of the system, a fail signal is generated.

24. An autoclave according to any foregoing claim wherein one of the control means and the monitoring meansis arranged to transmit to the other data relating to measurements of a parameter of the autoclave's operation.

25. An autoclave according to claim 24 wherein, if there is a disagreement between the control means and the monitoring means relating to a measured parameter, then a fail signal is generated.

26. An autoclave according to claim 24 or claim 25 when dependent on claim 11 wherein the control system is arranged to receive the signals from the temperature sensor and the pressure sensor, to determine whether the relationship between the two signals meets a predetermined condition, and produce an error output if the condition is not met.

27. An autoclave comprising an enclosure defining a chamber, steam supply means arranged to supply steam to the chamber, a temperature sensor arranged to produce a signal that varies with the temperature in the chamber, a pressure sensor arranged to produce a signal that varies with the pressure in the chamber and control means arranged to receive the signals from the temperature sensor and the pressure sensor, to determine whether the relationship between the two signals meets a predetermined condition, and produce an error output if the condition is not met.

28. An autoclave according to claim 26 or claim 27 wherein the condition depends on the expected relationship between the temperature and pressure of saturated steam.

29. In an autoclave having an enclosure defining a chamber and steam supply means arranged to supply steam to the chamber, a method of adapting a control system to control the operation of the autoclave comprises the steps of:
a) arranging the control system to have a control means and a monitoring means;
b) arranging each of the control means and the monitoring means to define respective sets of conditions required for a change of state of the autoclave; and
c) allowing the change of state for the autoclave only if both sets of conditions have been met.

30. Method of claim 29, further comprising the step of:
entering into a fail state if both sets of conditions are not met within a predetermined time.

31. In an autoclave having an enclosure defining a chamber, steam supply means arranged to supply steam to the chamber, and a plurality of sensors for sensing respective operating conditions of the autoclave, a method of determining whether the autoclave is operating correctly comprises the steps of:
utilizing a temperature sensor to produce as one of the conditions a signal that varies with the temperature in the chamber;
utilizing a pressure sensor to produce as another of the conditions a signal that varies with the pressure in the chamber;
sending the signals from the temperature sensor and the pressure sensor to a controller;
arranging the controller to determine whether the relationship between the two signals meets a predetermined condition; and
producing an error output if the condition is not met.
